Europäisches Patentamt

European Patent Office

Office européen des brevets

(11) Publication number: **0 248 074 B1**

(12) # EUROPEAN PATENT SPECIFICATION

(45) Date of publication of patent specification: **27.06.90**

(21) Application number: **87900398.6**

(22) Date of filing: **24.11.86**

(86) International application number:
**PCT/US86/02511**

(87) International publication number:
**WO 87/03280 04.06.87 Gazette 87/12**

(51) Int. Cl.⁵: **C 07 C 67/36, C 07 C 69/76, C 07 C 19/07, C 07 C 17/16**

(54) **PROCESS FOR THE CO-PRODUCTION OF AROMATIC CARBOXYLATES AND ALKYL IODIDES.**

(30) Priority: **26.11.85 US 801902**
**24.10.86 US 922574**

(43) Date of publication of application:
**09.12.87 Bulletin 87/50**

(45) Publication of the grant of the patent:
**27.06.90 Bulletin 90/26**

(84) Designated Contracting States:
**DE FR GB IT NL**

(56) References cited:
**EP-A-0 165 168**
**US-A-3 636 082**
**US-A-3 988 358**

(73) Proprietor: **EASTMAN KODAK COMPANY (a New Jersey corporation)**
**343 State Street**
**Rochester New York 14650 (US)**

(72) Inventor: **RULE, Mark**
**5625 Apache Drive**
**Kingsport, TN 37664 (US)**
Inventor: **LARKINS, Thomas, Hassell, Jr.**
**Route 8, 4408 Beechcliff Drive**
**Kingsport, TN 37664 (US)**
Inventor: **LANE, Donald, Wayne**
**605 Wessex Drive**
**Kingsport, TN 37663 (US)**
Inventor: **STEINMETZ, Guy, Ralph**
**835 Sir Echo Drive**
**Kingsport, TN 37663 (US)**

(74) Representative: **Buff, Michel et al**
**Kodak-Pathé Département des Brevets et Licences CRT Centre de Recherches et de Technologie Zone Industrielle**
**F-71102 Chalon sur Saône Cédex (FR)**

Courier Press, Leamington Spa, England.

## Description

This invention relates to a novel carbonylation process for the preparation of both aromatic carboxylic acids or esters and an iodine containing compound from which the iodine values can be economically recovered. The carbonylation is conducted in the presence of an alkanol and a catalytic amount of palladium.

The carbonylation of aromatic halides in the presence of palladium to obtain aromatic carboxylic acids and esters is well known in the art. U.S. Patent 3,988,358 discloses the carbonylation of aromatic halides in the presence of an alcohol and a tertiary amine to produce the corresponding carboxylic acid ester.

While it is known that aromatic iodides can be carbonylated the use of these materials has been discouraged by the cost associated with the difficulty of recovering the iodine values. For example, the use of basic materials in the carbonylation of aromatic halides, such as tri-n-butyl amine in U.S. 3,988,358, results in the formation of halide salts from which the halide values can be reclaimed only through uneconomical procedures involving severe chemical treatments.

We have discovered a process which not only results in the carbonylation of aromatic iodides to aromatic carboxylic acids or esters in excellent yields and at excellent rates of conversion but also results in production of alkyl iodides from which the iodine values can be economically recovered. In this invention the carbonylation is conducted in the presence of an alkanol and a catalytic amount of a palladium catalyst under aromatic carboxylic ester and alkyl iodide-forming conditions of temperature and pressure. The advantage afforded by our invention over the prior art is that the iodine values in the alkyl iodide may be readily recovered by simply flashing the relatively volatile alkyl iodide from the mixture resulting from the carbonylation reaction. This can be accomplished either in the carbonylation reactor or, more preferably, in a pressure reduction vessel to which the mixture resulting from the carbonylation reaction is fed.

The process of this invention can be thought of as a process for the co-production of aromatic carboxylic esters and alkyl iodides which comprises carbonylating aromatic iodides in the presence of an alkanol and a catalytic amount of a palladium catalyst under aromatic carboxylic ester and alkyl iodide-forming conditions of temperature and pressure.

The ratio of aromatic acids to esters produced in the present invention is dependent on the ratio of alkanol to water present in the carbonylation reactor and on the choice of organic co-solvent. In general, minimizing the ratio of alkanol to water maximizes the production of acid. Conversely maximizing the ratio of alkanol to water maximizes the production of ester.

The aromatic iodides which may be used in our process may be monoiodo or polyiodo, e.g. di-, tri- and tetra-iodo aromatic compounds. The aromatic nucleus or moiety can contain from 6 to 18 carbon atoms, preferably 6 to 10 carbon atoms and may be carbocyclic aromatic such as benzene, biphenyl, terphenyl, naphthalene, anthracene, etc., or heterocyclic aromatic such as pyridine, thiophene, pyrrole, indole, etc. In addition to one or more iodine atoms, the aromatic moiety may be substituted by various substituents inert under the conditions employed in our process. Examples of such substituents include alkyl of up to 12 carbon atoms such as methyl, ethyl, isobutyl, hexyl, 2-ethylhexyl, nonyl, decyl, dodecyl, etc.; cycloalkyl of 5 to 12 carbon atoms such as cyclopentyl, cyclohexyl, 4-butylcyclohexyl, etc.; hydroxy; alkoxy of up to 12 carbon atoms such as methoxy, ethoxy, propoxy, butyoxy, octyloxy, etc.; halogen such as chloro and bromo; alkoxycarbonyl of from 2 to 8 carbon atoms such as methoxycarbonyl, ethoxycarbonyl, butoxycarbonyl, hexyloxycarbonyl, etc.; carboxyl; cyano; alkenyl of 2 to 12 carbon atoms such as vinyl, allyl, etc.; formyl; alkanoyl of 2 to 8 carbon atoms such as acetyl, propionyl, butyryl, hexanoyl, etc.; alkanoylamido of 2 to 8 carbon atoms such as acetamido, butylamido, etc.; aroylamino such as benzamido; and alkylsulfonamide such as methanesulfonamide, hexanesulfonamido, etc.

Specific examples of the aromatic iodide reactants include iodobenzene, 1,3- and 1,4-diodobenzene, 1,3,5-triiodobenzene, 4-iodotoluene, 4-iodophenol, 4-iodoanisole, 4-iodoacetophenone, 4,4'-diiodobiphenyl, 4-chloroiodobenzene, 3-bromoiodobenzene, and 2,6- and 2,7-diiodonaphthalene. Our process is particularly useful for the preparation of benzenedicarboxylic and naphthalenedicarboxylic acids and their esters and thus the preferred reactants are diiodobenzenes, especially 1,3- and 1,4-diiodobenzene, and diiodonaphthalenes, especially 2,6- and 2,7-diiodonaphthalene.

The aromatic iodide reactants are known compounds and/or can be prepared according to published procedures. For example, T. Hudlicky et al *The Chemistry of Halides, Pseudohalides and Azides,* 1983 Supplement D, Part 2, 1142—1158, the disclosure of which is incorporated herein by reference in its entirety, discloses a number of such processes. Another process described in J. Chem. Soc. 150 (1952) comprises treating an aromatic compound, such as benzene, with iodine in the presence of silver sulfate dissolved in concentrated sulfuric acid.

The alkanol used in the process of this invention normally is methanol since it is the least expensive, results in the formation of methyl carboxylate esters, which may be used in transesterification reactions, and produces methyl iodide which is the most volatile of the alkyl iodides. However, other alkanols, for example, alkanols containing up to 12 carbon atoms, preferably up to 4 carbon atoms, may be employed if desired. Examples of such alkanols include ethanol, propanol, butanol, pentanol, hexanol, heptanol, octanol, decanol, ethylene glycol, diethylene glycol, benzyl alcohol, and the like. If aromatic esters are desired, two moles of alkanol are required for each mole equivalent of iodoaromatic reacting. For each mole equivalent of aromatic acid produced, one mole of alkanol is required.

2

The process provided by our invention can also be carried out in the presence of an organic co-solvent such as aliphatic, alicyclic and aromatic hydrocarbons, halogenated hydrocarbons and ethers. Examples of such inert solvents include benzene, toluene, the xylenes, hexane, heptane, chlorobenzene, ethylene dichloride, methylchloroform, diethyl ether, methyl t-butyl ether, diglyme, acetic acid, benzoic acid, methyl benzoate, etc. However, the use of a co-solvent is not critical to the practice of this invention.

The palladium catalyst can be provided to the reaction medium as either palladium metal or as any of a number of palladium salts or complexes, such as palladium acetate. The amount of palladium is not significant as long as enough is present to catalyze the reaction. Preferably, the catalyst is present in a concentration of 1 to 0.0001 mole percent, preferably 0.025 to 0.001 mole percent, based on the moles of aromatic iodide reactant. Therefore, the total reaction medium has a catalyst concentration of 1000 ppm to 0.1 ppm with preferred catalyst concentrations of 250 to 1 ppm.

The carbonylation reaction is conducted in the presence of carbon monoxide, which is employed in amounts such that the total reaction pressure is suitable for the formation of both the aromatic carboxylic ester and the alkyl iodide. The carbon monoxide employed may be essentially pure or it may contain other gases such as carbon dioxide, hydrogen, methane and other compounds produced by synthesis gas plants. Normally, the carbon monoxide will be at least 90, preferably at least 95, percent pure.

The process of the present invention can be conducted at temperatures and pressures suitable for formation of both the aromatic carboxylic acid and alkyl iodide. The temperatures and pressures are interdependent and can vary considerably. Normally, the pressure will be at least 690 kPa (7.0 kg/cm$^2$). While the process can be carried out at pressures as high as 69,000 kPa (703.1 kg/cm$^2$). The cost of utilities and equipment required for such high pressure operation cannot normally be commercially justified. Thus, the pressure normally will be in the range of 860 to 69,000 kPa (8.7 to 703.1 kg/cm$^2$), preferably 2,000 to 900 kPa (21.1 to 70.3 kg/cm$^2$). A particularly preferred pressure is 5,200 to 6,900 kPa (52.7 to 70.3 kg/cm$^2$). While temperatures as low as 125°C and higher than 225°C may be used, our process normally is carried out between 125 to 225°C. The preferred temperature range is 150 to 200°C.

The relative amounts of carbon monoxide, alkanol and aromatic iodide used in our process can be varied substantially and are, in general, not critical as long as there is at least a stoichiometric amount present.

When a polyiodo aromatic compound is used as the reactant in our carbonylation process, the products obtained include both aromatic polycarboxylic ester and partially carbonylated products such as iodo aromatic carboxylic esters. The latter compounds are useful as intermediates in the preparation of derivatives of aromatic carboxylic esters, for example, by displacement reactions whereby the iodo substituent is replaced with other radicals. The difunctional esters, such as dimethyl 2,6-naphthalene dicarboxylate, can be reacted with diols to produce high molecular weight polyesters suitable for molding plastics. Useful articles can be molded from these plastics, such as by injection molding. The relative amounts of partially or totally carbonylated products is highly dependent on the period of time that the reactant resides under carbonylation conditions. For example, the carbonylation of diiodobenzene at 175°C and 5,200 kPa (52.7 kg/cm$^2$) in accordance with our invention over varying periods of time results in varying amounts of reactant, iodo ester and diester as shown below:

|  | Reaction mixture | | |
|---|---|---|---|
| Carbonylation time, minutes | Diiodobenzene | Iodobenzoic ester | Benzene-dicarboxylic diester |
| 30 | 69 | 28 | 3 |
| 60 | 37 | 43 | 19 |
| 90 | 12 | 34 | 54 |
| 120 | 4 | 19 | 77 |

The alkyl iodides prepared according to the process of our invention may be used in other chemical processes such as in the preparation of carboxylic acids and carboxylic anhydrides according to known carbonylation procedures. Alternatively, the alkyl iodide can be oxidatively decomposed at elevated temperature to produce a gaseous mixture of iodine, carbon dioxide and water from which the iodine can be recovered. Alternatively, the alkyl iodides may be thermally decomposed to iodine and an alkane.

In our process there are no significant amounts of basic materials which preferentially combine with hydrogen iodide and interfere with the formation of an alkyl iodide. Examples of such bases which are not present in significant amounts in our process include amines, particularly tertiary amines, and hydroxides, alkoxides and weak acid salts, e.g. carboxylates, of the alkali and alkaline earth metals.

Our process is particularly useful for the preparation of dialkyl esters of aromatic dicarboxylic acids such as 1,3- and 1,4-benzene-dicarboxylic and 2,6- and 2,7-naphthalene-dicarboxylic acid esters. Such diesters may be used in the preparation of polyesters such as poly(ethylene terephthalate) and poly(ethylene 2,6-naphthalenedicarboxylate).

The process of this invention can be carried out as a batch, semi-continuous or continuous operation. In the manufacture of dialkyl esters of aromatic dicarboxylic acids in the quantities required for use in the preparation of polyesters such as those mentioned above, the process described hereinabove will be carried out in a continuous manner. A typical continuous method of practicing our process comprises feeding into a mixed pressure vessel a liquid stream of methanol, another liquid stream composed of 2,6-diiodonaphthalene, optionally an organic solvent and the palladium catalyst and a gaseous stream of carbon monoxide. The pressure vessel is equipped with a means for maintaining the desired temperature and pressure. The liquid mixture from the reactor is passed to a flash column where the methyl iodide and inert organic solvent is flashed off. The flashed vapor stream in then condensed and the methyl iodide and methanol separated by decanting. The liquid from the flash column is centrifuged and 2,6-naphthalene dicarboxylic acid and palladium are separated from the solution containing the ester of 2,6-naphthalene dicarboxylic acid. The desired 2,6-naphthalene dicarboxylic ester is then recovered by selective recrystallization and the remaining mixture containing unreacted iodoaromatics is recycled.

Our invention is further illustrated by the following examples. In the procedures utilized in the examples the materials employed are loaded into a 330 ml autoclave constructed of Hastelloy B2 alloy which is designed to operate in a rocking mode. The autoclave is pressurized with 3,500 kPa (35.2 kg/cm$^2$) carbon monoxide gas pressure at room temperature and then the gas is vented and the autoclave is sealed. In these examples the autoclave is pressurized to 14.0 kg/cm$^2$ with carbon monoxide gas at ambient temperature and heated and rocked until reaction temperature was reached, at which time additional carbon monoxide gas is added to increase the autoclave internal pressure to the predetermined value. Reactor pressure is maintained by adding carbon monoxide at the same rate at which it is consumed by the reactants. The carbon monoxide used is essentially pure. When the predetermined reaction time is completed the autoclave is cooled by a stream of cold air to approximately 25°C. After the gas is vented from the autoclave the crude product is isolated by filtration and analyzed by gas chromatographic methods. The % conversion is the mole percent of iodo-group converted to carboxylic acid or ester. The ester/acid ratio is the mole ratio of total ester and acid groups formed. The grams of alkyl iodide found were determined by gas chromatographic analysis of the reaction solution. The results of these runs are shown below.

| | | Example No. | |
|---|---|---|---|
| | | 1 | 2 |
| Iodoaromatic wt (g) | | p-diiodobenzene 60 | p,p'-diiodobiphenyl 50 |
| Alkanol wt (g) | | methanol 38 | methanol 30 |
| Co-Solvent wt (g) | | toluene 86 | toluene 80 |
| H$_2$O (g) | | — | — |
| Catalyst wt Pd(mg) | | Pd(OAc)$_2$ 2.0 | Pd(OAc)$_2$ 1.0 |
| Time (Min) | | 110 | 180 |
| Pressure (kg/cm$^2$) | | 52.7 5,200 kPa | 70.3 |
| Temp (°C) | | 150 | 200 |
| % Conversion | | 100 | 100 |
| Ester/Acid | | 24 | 25 |
| g. Alkyl Iodide | | 49 | 33 |

| | Example No. | |
| --- | --- | --- |
| | 3 | 4 |
| Iodoaromatic wt (g) | 2,6-diiodo-naphthalene 40 | 2,6-diiodo-naphthalene 40 |
| Alkanol wt (g) | methanol 38 | methanol 38 |
| Co-Solvent wt (g) | toluene 82 | toluene 86 |
| $H_2O$ (g) | 3 | — |
| Catalyst wt Pd(mg) | Pd(OAc)$_2$ 2.0 | Pd(OAc)$_2$ 2.0 |
| Time (Min) | 120 | 300 |
| Pressure (kg/cm$^2$) | 52.7 5,200 kPa | 52.7 |
| Temp (°C) | 150 | 150 |
| % Conversion | 100 | 100 |
| Ester/Acid | 6 | 9 |
| g. Alkyl Iodide | 28 | 28 |

| | Example No. | |
| --- | --- | --- |
| | 5 | 6 |
| Iodoaromatic wt (g) | 2,6-diiodo-naphthalene 40 | 2,6-diiodonaphthalene 40 |
| Alkanol wt (g) | methanol 38 | methanol 38 |
| Co-Solvent wt (g) | toluene 86 | toluene 86 |
| $H_2O$ (g) | — | — |
| Catalyst wt Pd(mg) | Pd(OAc)$_2$ 2.0 | Pd(OAc)$_2$ 2.0 |
| Time (Min) | 30 | 120 |
| Pressure (kg/cm$^2$) | 105.4 10,500 kPa | 35.2 |
| Temp (°C) | 150 | 160 |
| % Conversion | 100 | 100 |
| Ester/Acid | 17 | 16 |
| g. Alkyl Iodide | 28 | 28 |

| | Example No. | |
|---|---|---|
| | 7 | 8 |
| Iodoaromatic wt (g) | p-diiodobenzene 33 | iodobenzene 60 |
| Alkanol wt (g) | methanol 32 | methanol 39 |
| Co-Solvent wt (g) | toluene 43 | toluene 86 |
| $H_2O$ (g) | 40 | — |
| Catalyst wt Pd(mg) | $Pd(OAc)_2$ 0.47 | 5% Pd-C 50 |
| Time (Min) | 120 | 120 |
| Pressure $(kg/cm^2)$ | 52.7 5,200 kPa | 52.7 |
| Temp (°C) | 175°C | 175 |
| % Conversion | 100 | 100 |
| Ester/Acid | 0.08 | 8 |
| g. Alkyl Iodide | 27 | 39 |

| | Example No. | |
|---|---|---|
| | 9 | 10 |
| Iodoaromatic wt (g) | iodobenzene 60 | iodobenzene 60 |
| Alkanol wt (g) | methanol 112 | methanol 38 |
| Co-Solvent wt (g) | — — | toluene 85 |
| $H_2O$ (g) | — | — |
| Catalyst wt Pd(mg) | $Pd(OAc)_2$ 2.0 | $Pd(OAc)_6$ 39 |
| Time (Min) | 180 | 48 |
| Pressure $(kg/cm^2)$ | 52.7 5,200 kPa | 52.7 |
| Temp (°C) | 150 | 140 |
| % Conversion | 100 | 100 |
| Ester/Acid | 6 | 11 |
| g. Alkyl Iodide | 39 | 39 |

|  | Example No. | |
|---|---|---|
|  | 11 | 12 |
| Iodoaromatic wt (g) | p-diiodobenzene 20 | p-diiodobenzene 20 |
| Alkanol wt (g) | ethanol 75 | methanol 24 |
| Co-Solvent wt (g) | — — | hexane 43 |
| $H_2O$ (g) | — | — |
| Catalyst wt Pd(mg) | Pd(OAc)$_2$ 2.4 | Pd(OAc)$_2$ 2.4 |
| Time (Min) | 180 | 180 |
| Pressure (kg/cm$^2$) | 52.7 5,200 kPa | 52.7 |
| Temp (°C) | 175 | 175 |
| % Conversion | 100 | 100 |
| Ester/Acid | 3.2 | 18 |
| g. Alkyl Iodide | 16 | 16 |

|  | Example No. | |
|---|---|---|
|  | 13 | 14 |
| Iodoaromatic wt (g) | p-diiodobenzene 300 | p-iodophenol 50 |
| Alkanol wt (g) | methanol 142 | methanol 38 |
| Co-Solvent wt (g) | acetic acid 755 | toluene 86 |
| $H_2O$ (g) | — | — |
| Catalyst wt Pd(mg) | Pd(OAc)$_2$ 7.7 | Pd(OAc)$_2$ 2.0 |
| Time (Min) | 300 | 140 |
| Pressure (kg/cm$^2$) | 52.7 5,200 kPa | 52.7 |
| Temp (°C) | 175 | 150 |
| % Conversion | 100 | 100 |
| Ester/Acid | 0.09 | 16 |
| g. Alkyl Iodide | 244 | 30 |

# EP 0 248 074 B1

## Claims

1. A process for producing a mixture of an aromatic carboxylic ester and an aromatic carboxylic acid and an alkyl iodide by carbonylating an aromatic iodide or an heterocyclic aromatic iodide in the absence of basic materials and in the presence of an alkanol and a catalytic amount of a palladium catalyst wherein the temperature is in the range of about 125 to 225°C and the pressure in the range of 860 to 69,000 kPa (125 to 10,000 psig).

2. The process of claim 1 wherein the aromatic iodide is selected from the group consisting of diiodonaphthalene and diiodobenzenes.

3. The process of claim 2 wherein the diiodonaphthalene is 2,6-diiodonaphthalene and the diiodobenzene is 1,4-diiodobenzene.

4. The process of claim 1 wherein the alkanol is methanol.

5. The process of claim 1 wherein the temperature is in the range of about 150—200°C.

6. The process of claim 1 wherein the pressure is in the range of 2,000 to 6,900 kPa (300 to 1,000 psig).

7. The process of claim 1 wherein the process is carried out in the presence of an organic co-solvent.

8. The process of claim 1 comprising producing a mixture of an aromatic dicarboxylic ester selected from the group consisting of dimethyl benzenedicarboxylate and dimethyl naphthalenedicarboxylate and methyl iodide by carbonylating a diiodoaromatic compound selected from the group consisting of a diiodobenzene or a diiodonaphthalene in the absence of basic materials and in the presence of methanol, an organic co-solvent and a catalytic amount of a palladium catalyst at a temperature of about 150 to 200°C and a pressure of about 2,000 to 6,900 kPa (300 to 1,000 psig).

9. The process of claim 1 comprising producing a mixture of dimethyl 2,6-naphthalenedicarboxylate and methyl iodide by carbonylating 2,6-diiodonaphthalene in the absence of basic materials and in the presence of methanol, an organic co-solvent and a catalytic amount of palladium at a temperature of about 175°C and a pressure of about 5,200 kPa (750 psig).

## Patentansprüche

1. Verfahren zur Herstellung einer Mischung aus einem aromatischen Carbonsäureester und einer aromatischen Carbonsäure sowie einem Alkyliodid durch Carbonylierung eines aromatischen Iodides oder eines heterocyclischen aromatischen Iodides in Abwesenheit basischer Stoffe und in Anwesenheit eines Alkanols sowie einer katalytisch wirksamen Menge eines Palladiumkatalysators, bei einer Temperatur im Bereich von etwa 125 bis 225°C sowie einem Druck im Bereich von 860 bis 69 000 kPa.

2. Verfahren nach Anspruch 1, in dem das aromatische Iodid ausgewählt ist aus der Gruppe bestehend aus Diiodonaphthalinen und Diiodobenzolen.

3. Verfahren nach Anspruch 2, in dem das Diiodonaphthalin aus 2,6-Diiodonaphthalin und das Diiodobenzol aus 2,4-Diiodobenzol besteht.

4. Verfahren nach Anspruch 1, in dem das Alkanol aus Methanol besteht.

5. Verfahren nach Anspruch 1, in dem die Temperatur im Bereich von etwa 150—200°C liegt.

6. Verfahren nach Anspruch 1, in dem der Druck im Bereich von 2 000 bis 6 900 kPa liegt.

7. Verfahren nach Anspruch 1, das in Gegenwart eines organischen Co-Lösungsmittels durchgeführt wird.

8. Verfahren nach Anspruch 1, in dem man eine Mischung aus einem aromatischen Dicarbonsäureester, ausgewählt aus der Gruppe bestehend aus Dimethylbenzoldicarboxylat und Dimethylnaphthalindicarboxylat sowie Methyliodid durch Carbonylierung einer diiodoaromatischen Verbindung, ausgewählt aus der Gruppe bestehend aus Diiodobenzol oder einem Diiodonaphthalin in Abwesenheit basischer Stoffe und in Gegenwart von Methanol, einem organischen Co-Lösungsmittel und einer katalytisch wirksamen Menge eines Palladiumkatalysators bei einer Temperatur von etwa 150 bis 200°C und einem Druck von etwa 2000 bis 6 900 kPa herstellt.

9. Verfahren nach Anspruch 1, bei dem man eine Mischung aus Dimethyl-2,6-naphthalindicarboxylat und Methyliodid durch Carbonylierung von 2,6-Diiodonaphthalin in Anwesenheit basischer Stoffe und in Gegenwart von Methanol, einem organischen Co-Lösungsmittel und einer katalytisch wirksamen Menge Palladium bei einer Temperatur von etwa 175°C und einem Druck von etwa 5 200 kPa herstellt.

## Revendications

1. Procédé pour produire un mélange d'un ester carboxylique aromatique et d'un acide carboxylique aromatique et un iodure d'alkyle par carbonylation d'un iodure aromatique ou d'un iodure aromatique hétérocyclique en l'absence de substances basiques et en présence d'un alcanol et d'une quantité catalytique de catalyseur a base de palladium dans lequel la température est comprise entre 125 et 225°C et la pression est comprise entre 860 et 69 000 kPa (125 à 10 000 psig).

2. Procédé selon la revendication 1, dans lequel l'iodure aromatique est choisi dans le groupe comprenant le diiodonaphtalène et les diiodobenzènes.

3. Procédé selon la revendication 2, dans lequel le diiodonaphtalène est le 2,6-diiodonaphtalène et le diiodobenzène est le 1,4-diiodobenzène.

8

4. Procédé selon la revendication 1, dans lequel l'alcanol est le méthanol.

5. Procédé selon la revendication 1, dans lequel la température est comprise entre 150 et 200°C.

6. Procédé selon la revendication 1, dans lequel la pression est comprise entre 2000 et 6900 kPa (300 à 1000 psig).

7. Procédé selon la revendication 1, dans lequel le procédé est réalisé en présence d'un cosolvant organique.

8. Procédé selon la revendication 1, consistant à produire un mélange d'un ester dicarboxylique aromatique choisi parmi le groupe comprénant le diméthyl benzène dicarboxylate et le diméthyl naphtalène dicarboxylate et un iodure de méthyle par carbonylation d'un composé diiodoaromatique choisi parmi le groupe comprenant un diiodobenzène ou un diiodonaphtalène en l'absence de substances basiques et en présence de méthanol, d'un cosolvant organique et d'une quantité catalytique de catalyseur à base de palladium à une température comprise entre environ 150 et 200°C et une pression comprise entre environ 2000 et 6900 kPa (300 à 1000 psig).

9. Procédé selon la revendication 1, consistant à produire un mélange de diméthyl 2,6-naphtalène dicarboxylate et d'iodure de méthyle par carbonylation de 2,6-diiodonaphtalène en l'absence de substances basiques et en présence de méthanol, d'un cosolvant organique et d'une quantité catalytique de palladium à une température d'environ 175°C et une pression d'environ 5200 kPa (750 psig).